# FASCICULE DE BREVET EUROPEEN

(11) **EP 4 292 621 B1**
(45) Date de publication et mention de la délivrance du brevet: **19.03.2025**
(21) Numéro de dépôt: 23178819.1
(22) Date de dépôt: 13.06.2023
(51) Int. Cl.: A61M 1/02, A61M 1/36

(54) **DISPOSITIF DE MAINTIEN POUR UNITE DE FILTRATION D'UN FLUIDE BIOLOGIQUE**
HALTEVORRICHTUNG FÜR EINE FILTEREINHEIT FÜR EINE BIOLOGISCHE FLÜSSIGKEIT
HOLDING DEVICE FOR A BIOLOGICAL FLUID FILTRATION UNIT

(30) Priorité: 14.06.2022 FR 2205706
(43) Date de publication de la demande: 20.12.2023
(73) Titulaire: Maco Pharma, 59420 Mouvaux (FR)
(72) Inventeur: Legrand, Nicolas, 74330 Poisy (FR); Houssin, Timothée, 59420 Mouvaux (FR); Paquet, Louis, 59700 Marcq-en-Baroeul (FR)
(74) Mandataire: Sayettat, Julien Christian

(56) Documents cités:
- EP-A1- 0 919 249
- WO-A1-2018/122105
- WO-A2-03/033066
- CN-A- 114 452 209
- JP-A- 2001 149 444

## Description

La présente invention concerne un dispositif de maintien d'une unité de filtration ainsi qu'un ensemble comprenant ledit dispositif de maintien et ladite unité de filtration.

L'invention s'applique au domaine des dispositifs médicaux et notamment aux dispositifs médicaux destinés à la filtration du sang ou d'un composant sanguin.

Le sang total est constitué de deux types de composants : les cellules sanguines comprenant les globules rouges, les leucocytes et les plaquettes, et le plasma, liquide jaune pâle dans lequel les cellules sanguines sont en suspension. Actuellement, ne sont transfusés que les composants sanguins nécessaires aux patients.

Dans les centres de traitement du sang, ces différents composants sanguins sont généralement obtenus par centrifugation : le sang total d'un donneur est recueilli dans une poche dite primaire d'un système à poches. Puis, le système à poches est placé dans une centrifugeuse pour séparer le sang contenu dans la poche primaire en composants sanguins tels qu'un concentré de globules rouges (CGR) et, soit un plasma riche en plaquettes (PRP), soit un plasma pauvre en plaquettes (PPP) et une couche leuco-plaquettaire.

Le système à poches est ensuite placé dans un appareil de séparation et d'extraction des composants sanguins tel que celui décrit dans le document EP 2 869 864 A1. Dans cet appareil de séparation, la poche primaire contenant les composants sanguins séparés est comprimée de manière à extraire par exemple le concentré de globules rouges de la poche primaire vers une première poche satellite et le plasma de la poche primaire vers une deuxième poche satellite du système à poches.

En outre, dans le champ de la transfusion, il est classique de traiter le sang ou les composants sanguins pour en éliminer les substances indésirables telles que les leucocytes, les pathogènes, certaines protéines - par exemple le prion -, et/ou les substances utilisées dans des procédés d'inactivation, de réduction et/ou d'élimination des pathogènes.

Pour éliminer ces substances indésirables par filtration, on utilise une unité de filtration dans laquelle un milieu de filtration apte à éliminer les substances indésirables du fluide est enfermé.

Ces unités de filtration sont généralement intégrées dans les systèmes à poches décrits ci-dessus permettant de traiter le sang et les composants sanguins en circuit clos.

Il existe deux grandes catégories d'unités de filtration : les unités de filtration dites souples comprenant une enveloppe extérieure souple, et les unités de filtration dites rigides comprenant un boîtier rigide.

Avec des unités de filtration rigides, durant l'étape de centrifugation pour séparer les composants sanguins, il existe un risque non seulement que les unités de filtration rigides soient endommagées mais encore que ces unités de filtration rigides endommagent les poches adjacentes, rendant inutilisables les systèmes à poches et contaminant possiblement l'environnement extérieur et l'utilisateur.

Les unités de filtration souples, comme celle du document EP-A-526 678, permettent d'éviter la détérioration des systèmes à poches durant la centrifugation. Mais, avec ce type d'unités de filtration souples, lors de la filtration, une chute de pression se produit due à la résistance du milieu de filtration, conduisant au gonflement du compartiment entrée de l'unité de filtration et augmentant la perte en fluide dans l'unité de filtration.

Enfin, on distingue également deux types de filtration : par gravité et sous pression. Par gravité, la filtration du sang ou des composants sanguins est réalisée en suspendant simplement le système à poches avec l'unité de filtration à une potence. Sous pression, la filtration est par exemple effectuée simultanément à l'extraction lors de la séparation par un appareil de séparation tel que décrit ci-dessus.

Dans le cas de la filtration du plasma, la filtration sous pression présente les avantages suivants : le système à poches présente une poche en moins puisque la poche de transfert de plasma avant filtration n'est plus nécessaire, la mise en place du système dans les pots de centrifugation est ainsi facilitée, le temps de filtration est plus court et l'utilisation plus simple puisqu'il n'est pas nécessaire de suspendre les poches à une potence.

Cependant, la filtration sous pression avec une unité de filtration souple augmente les inconvénients liés à ce type d'unité : la différence de pression due à la résistance du milieu de filtration est plus importante, ainsi que le gonflement de l'unité de filtration et la perte de sang associée. C'est pourquoi, des conformateurs pour unités de filtration ont été proposés.

Par exemple le document JP2001149444 décrit plusieurs réalisations de support rigide pour filtre souple permettant d'éviter le gonflement du filtre.

Dans une première réalisation du document JP2001149444, le support de filtre comprend deux parois plates parallèles entre elles et séparées d'une distance égale ou supérieure à l'épaisseur du filtre. Le support comprend en outre des encoches pour les tubes d'entrée et sortie du filtre. Avec ce type de support, il est difficile de glisser le filtre entre les deux parois, notamment lorsque l'épaisseur du filtre est substantiellement égale à la distance entre les deux parois. Et, en augmentant la distance entre les deux parois du support de filtre pour faciliter l'insertion du filtre, la compression du filtre devient insuffisante pour limiter le volume de perte de fluide dans le filtre.

Le document JP2001149444 fournit une autre réalisation de support dans laquelle les deux parois du support pivotent entre elles autour d'une charnière de sorte à pouvoir ouvrir et fermer le support. Un élément de fixation est pourvu sur chacune des parois pour fermer le support.

Le document WO 03/033066A2 divulgue également un support de filtre à charnière dont les parois plates sont maintenues dans une position fermée à l'aide d'un verrou amovible.

L'inconvénient avec ce type de support de filtre à charnière est le risque que l'élément de fixation des parois ne supporte pas la pression infligée par le gonflement du filtre lors d'une filtration sous pression et s'ouvre durant la filtration.

En effet, la pression de filtration est directement liée à la dénivellation entre la poche de sang à filtrer et la poche de sang filtré. Par exemple, pour une dénivellation de 1,2 m, la pression de filtration est de l'ordre de 0,12 bar. Dans un appareil de séparation et d'extraction, la pression de filtration peut être près de trois fois plus importante et peut atteindre environ 0,3 bar.

Le dispositif de maintien selon l'invention permet de remédier à ces inconvénients en fournissant un dispositif de maintien facile à utiliser et pouvant résister à des pressions de filtration élevées.

Selon un premier aspect, l'invention concerne un dispositif de maintien d'une unité de filtration d'un fluide biologique comprenant une première plaque et une deuxième plaque mobiles entre elles entre un état ouvert dans lequel la première plaque et la deuxième plaque sont éloignées l'une de l'autre afin de pouvoir disposer ladite unité de filtration dans ledit dispositif de maintien, et un état fermé dans lequel la première plaque et deuxième plaque sont en vis-à-vis afin de maintenir serrée ladite unité de filtration, ledit dispositif de maintien comprenant en outre un élément de fixation mobile entre une position libre dans laquelle la première plaque et la deuxième plaque sont libres de se déplacer entre l'état ouvert et l'état fermé et une position de blocage dans laquelle la première plaque et la deuxième plaque sont maintenues dans l'état fermé, ledit dispositif de maintien comprenant en outre un élément de verrouillage configuré pour maintenir l'élément de fixation dans ladite position de blocage.

Selon un deuxième aspect, l'invention porte sur un ensemble comprenant un dispositif de maintien selon le premier aspect de l'invention et une unité de filtration.

Les dessins annexés illustrent l'invention :
[Fig.1] représente une vue en perspective du dispositif de maintien de l'invention en état ouvert, dans lequel une unité de filtration est placée.
[Fig.2] représente une vue en perspective du dispositif de maintien de la figure 1 en état fermé et dans lequel une unité de filtration est placée.
[Fig.3] représente une vue en éclaté des différents éléments du dispositif de maintien de la figure 1.
[Fig.4] représente une vue en perspective et de dessous de l'élément de fixation du dispositif de maintien de la figure 1.
[Fig.5] représente une vue en perspective de la face externe de la première plaque du dispositif de maintien de la figure 1.
[Fig.6] représente une vue schématique d'un système à poches comprenant une unité de filtration pouvant être maintenue par le dispositif de maintien de la figure 1.

L'invention concerne un dispositif de maintien d'une unité de filtration d'un fluide biologique, tel que le sang ou un composant sanguin, notamment le plasma sanguin.

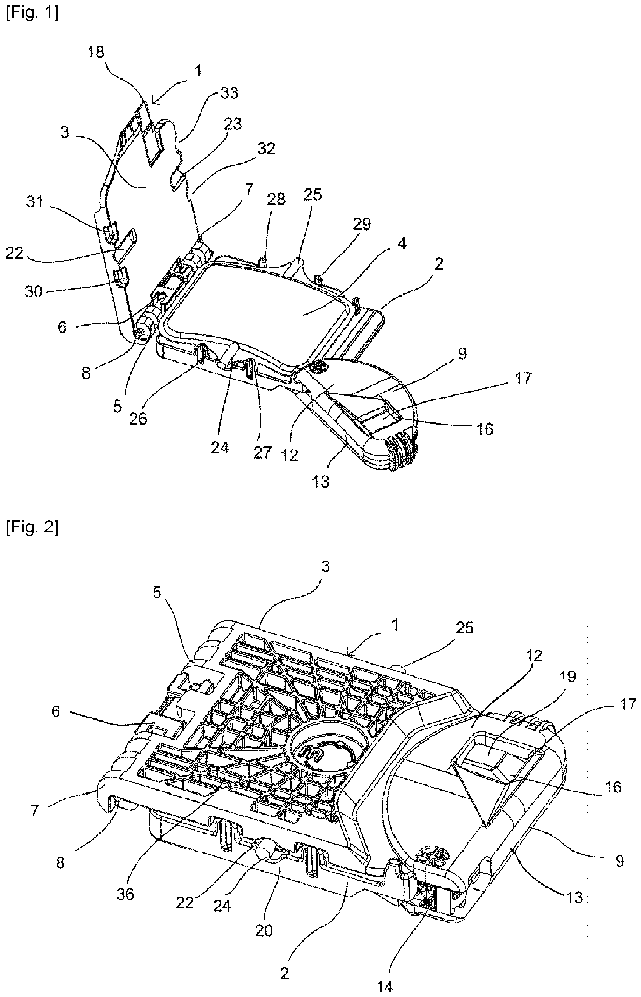

En lien avec les figures 1 à 3, le dispositif de maintien 1 comprend une première plaque 2 et une deuxième plaque 3 mobiles entre elles entre un état ouvert (figure 1) dans lequel la première plaque 2 et la deuxième plaque 3 sont éloignées l'une de l'autre afin de pouvoir disposer une unité de filtration 4 dans ledit dispositif de maintien 1, et un état fermé (figure 2) dans lequel la première plaque 2 et deuxième plaque 3 sont en vis-à-vis et maintiennent serrée ladite unité de filtration 4.

En particulier, l'unité de filtration 4 comprend une enveloppe souple renfermant un milieu de filtration destiné à retenir les substances indésirables du sang telles que les leucocytes. L'unité de filtration 4 comprend en outre un tube d'entrée 24 pour le fluide à filtrer et un tube de sortie 25 pour le fluide filtré.

Dans l'état ouvert, la première plaque 2 et la deuxième plaque 3 sont suffisamment éloignées l'une de l'autre pour pouvoir positionner facilement l'unité de filtration 4 sur la première plaque 2.

Dans l'état fermé, la première plaque 2 et la deuxième plaque 3 sont substantiellement parallèles entre elles.

Dans une configuration particulière, la première plaque 2 et la deuxième plaque 3 sont séparées d'une distance inférieure à l'épaisseur de l'unité de filtration 4. La distance entre la première plaque 2 et la deuxième plaque 3 est déterminée pour que, lorsque les plaques 2,3 sont dans l'état fermé, l'unité de filtration 4 est serrée pour éviter son gonflement mais pas totalement comprimée pour permettre la circulation du fluide à filtrer au travers de l'unité de filtration. La limitation du gonflement de l'unité de filtration qui survient lors de la filtration, réduit ainsi la perte en volume du fluide à filtrer.

Dans une configuration particulière, la première plaque 2 et la deuxième plaque 3 sont reliées l'une à l'autre par une articulation formée d'une charnière 5.

En particulier, la charnière 5 est formée d'une première partie cylindrique 6 arrangée sur la première plaque 2 et d'une deuxième partie cylindrique 7 arrangée sur la deuxième plaque 3, lesdites première et deuxième parties cylindriques 6,7 coopérant entre elles pour s'aligner entre elles. La première partie cylindrique 6 et la deuxième partie cylindrique 7 sont jointes par une broche 8 formant axe de rotation, qui traverse la première et deuxième partie cylindrique 6,7.

Le dispositif de maintien 1 comprend en outre un élément de fixation 9 mobile entre une position libre (figure 1) dans laquelle la première plaque 2 et la deuxième plaque 3 sont libres de se déplacer entre l'état ouvert et l'état fermé et une position de blocage (figure 2) dans laquelle la première plaque 2 et la deuxième plaque 3 sont maintenues dans l'état fermé.

Selon une configuration particulière et comme représenté sur les figures, l'élément de fixation 9 comprend une pièce présentant une fente 10 conçue pour recevoir au moins une portion de ladite première plaque 2 et de ladite deuxième plaque 3 lorsqu'elles sont dans l'état fermé.

Plus particulièrement, la pièce de l'élément de fixation 9 est formée d'une première plaquette 11 et d'une deuxième plaquette 12 se faisant face, reliées entre elles par un fond 13. Pour maintenir dans l'état fermé la première plaque 2 et la deuxième plaque 3, la première plaquette 11 et la deuxième plaquette 12 sont séparées d'une distance substantiellement égale à l'épaisseur de la première plaque 2 et de la deuxième plaque 3 dans l'état fermé avec l'unité de filtration maintenue serrée dans le dispositif de maintien.

Comme représenté sur les figures, l'élément de fixation 9 est configuré pour pivoter entre la position de blocage et la position libre. Pour cela, l'élément de fixation 9 comprend à une extrémité, une tige 14 formant axe de rotation. La tige 14 est arrangée à une extrémité de l'élément de fixation 9, entre la première plaquette 11 et la deuxième plaquette 12.

La première plaque 2 comprend un pivot 15 conçu pour supporter la tige 14 de l'élément de fixation 9 et permettre ainsi le pivotement dudit élément de fixation 9.

Le dispositif de maintien 1 comprend en outre un élément de verrouillage 16 configuré pour maintenir l'élément de fixation 9 dans la position de blocage illustrée sur la figure 2.

Selon une configuration particulière, l'élément de verrouillage 16 comprend une partie femelle formée d'un orifice 17 et une partie mâle formée d'une languette 18 pourvue d'un ergot 19, ladite partie femelle et ladite partie mâle coopérant pour que l'ergot 19 s'engage dans l'orifice 17 de la partie femelle.

Sur les figures, la partie femelle est arrangée sur la deuxième plaquette 12 de l'élément de fixation 9 et la partie mâle est arrangée sur la deuxième plaque 3 du dispositif de maintien 1.

Lorsque l'ergot 19 est engagée dans l'orifice 17, la partie femelle et la partie mâle de l'élément de verrouillage 16 sont verrouillées, plaçant l'élément de fixation 9 en position de blocage.

La languette 18 est déformable pour permettre le déverrouillage de l'élément de verrouillage 16 par pression sur ladite languette 18 afin de désengager l'ergot 19 de l'orifice 17.

Grâce à l'agencement particulier de cet élément de verrouillage 16, lorsque l'élément de fixation 9 passe de l'état ouvert à l'état fermé, l'élément de verrouillage 16 passe simultanément de la position libre à la position de blocage.

Avantageusement, l'élément de verrouillage 16 et l'unité de filtration 4 sont superposés lorsque l'unité de filtration est placée dans le dispositif de maintien dans l'état fermé. Ainsi, lors de la filtration, l'unité de filtration souple tend à gonfler et exerce une pression sur la languette 18 de l'élément de verrouillage 16. Cette pression assure que l'ergot 19 reste engagé dans l'orifice 17 tout au long de la filtration.

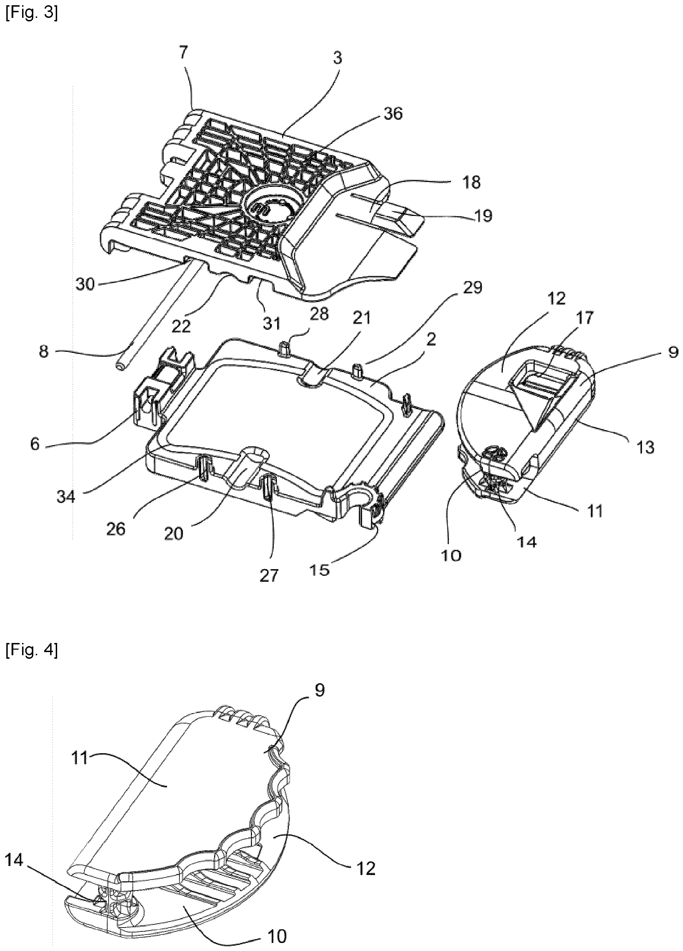

En lien avec les figures 1 et 3, chacune des première et deuxième plaques 2,3 comprennent sur leur face interne des cavités 20,21,22,23 configurées pour recevoir des tubes d'entrée 24 et sortie 25 de l'unité de filtration 4.

Les termes « interne » et « externe » sont définis par rapport au dispositif de maintien dans l'état fermé. La face interne d'une plaque est tournée vers l'intérieur du dispositif de maintien. La face externe d'une plaque est la face visible de l'extérieur.

Plus particulièrement, la première plaque 2 comprend une première cavité 20 et une deuxième cavité 21 conçues pour recevoir le tube d'entrée 24 et le tube de sortie 25 de l'unité de filtration 4, respectivement. La deuxième plaque 3 comprend une troisième cavité 22 et une quatrième cavité 23 conçues pour recevoir le tube d'entrée 24 et le tube de sortie 25 de l'unité de filtration 4, respectivement. La troisième cavité 22 et la quatrième cavité 23 sont en vis-à-vis avec la première cavité 20 et de la deuxième cavité 21, respectivement, lorsque le dispositif de maintien est dans l'état fermé.

Les cavités 20,21,22,23 permettent de maintenir serrée l'unité de filtration 4 entre la première plaque 2 et la deuxième plaque 3 sans comprimer les tubes d'entrée 24 et de sortie 25. Ainsi, le fluide s'écoule dans l'unité de filtration 4 même lorsque l'unité de filtration est maintenue serrée dans le dispositif de maintien.

Afin de faciliter la mise en place et la tenue de l'unité de filtration dans le dispositif de maintien, l'une des première plaque 2 ou deuxième plaque 3 comprend au moins une saillie 26,27,28,29 coopérant avec au moins une encoche 30,31,32,33 prévue sur l'autre de la première plaque 2 ou deuxième plaque 3.

Selon la figure 3, une première paire de saillies 26,27 est agencée sur la première plaque 2 et encadre la première cavité 20 prévue pour recevoir le tube d'entrée 24 de l'unité de filtration 4. La première plaque 2 comprend en outre une deuxième paire de saillies 28,29 encadrant la deuxième cavité 21 prévue pour recevoir le tube de sortie 25 de l'unité de filtration 4.

Une première paire d'encoches 30,31 encadre la troisième cavité 22 et fait face à la première paire de saillies 26,27. De plus, une deuxième paire d'encoches 32,33 encadrent la quatrième cavité 23 et fait face à la deuxième paire de saillies 28,29.

Avantageusement, la face interne de la première plaque 2 ou la deuxième plaque 3 comprend une empreinte 34 de l'unité de filtration 4. Cette empreinte 34 marque l'emplacement de l'unité de filtration 4 dans le dispositif de maintien 1 et aide l'utilisateur à positionner correctement l'unité de filtration 4.

Sur la figure 3, la face interne de la première plaque 2 comprend une empreinte 34 de l'unité de filtration.

Avantageusement, le dispositif de maintien 1 est transparent afin de visualiser l'écoulement du fluide dans l'unité de filtration.

Le dispositif de maintien est réalisé dans un matériau thermoplastique rigide tel que le polycarbonate ou un copolymère d'acrylonitrile butadiène styrène.

Selon une réalisation, les éléments du dispositif de maintien 1, à savoir la première plaque 2, la deuxième plaque 3 et l'élément de fixation 9, est réalisé par moulage par injection puis assemblés entre eux. La broche 8 est avantageusement en métal.

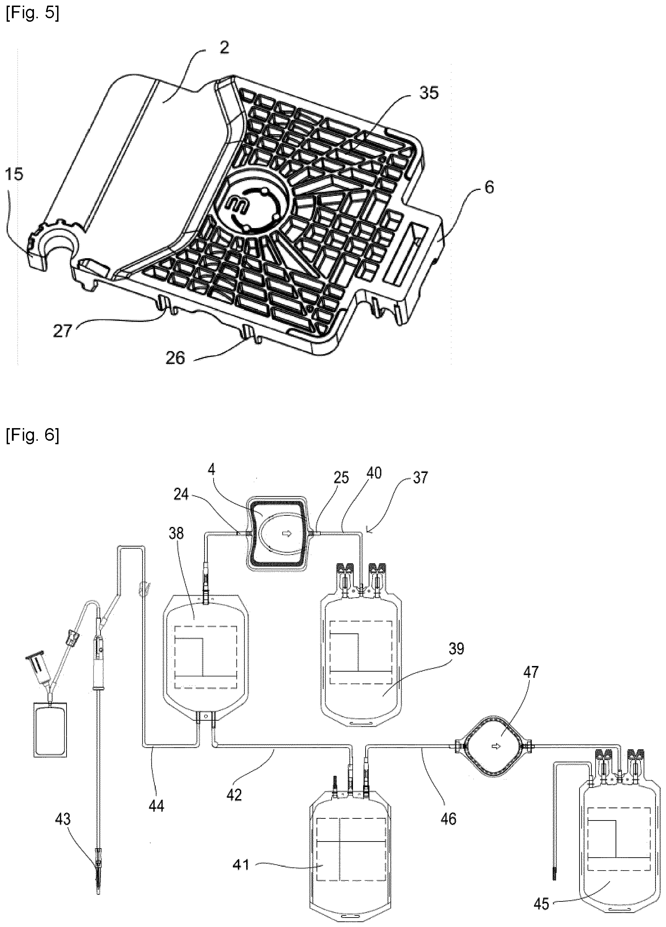

Comme montré sur les figures 2 et 5, les faces externes de la première plaque 2 et de la deuxième plaque 3 comprennent chacune une pluralité d'évidements 35,36, respectivement. Ces évidements assurent une bonne rigidité et résistance à la pression en minimisant la quantité de matière du dispositif de maintien.

On décrit ci-dessous l'utilisation du dispositif de maintien des figures 1 à 5.

Initialement, la première plaque 2 et la deuxième plaque 3 du dispositif de maintien 1 sont dans l'état fermé (figure 2). L'utilisateur appuie sur l'ergot 19 de l'élément de verrouillage 16 afin de déverrouiller l'élément de fixation 9 de sa position de blocage et fait simultanément pivoter l'élément de fixation 9 dans sa position libre. L'utilisateur fait ensuite tourner la deuxième plaque 3 autour de la charnière 5 pour mettre la première plaque 2 et la deuxième plaque 3 en l'état ouvert (figure 1). L'utilisateur place alors l'unité de filtration 4 sur l'empreinte 34 de la première plaque 2 et fait tourner à nouveau la deuxième plaque 3 pour mettre la première plaque 2 et la deuxième plaque 3 en état fermé, l'unité de filtration 4 étant dans ledit dispositif de maintien 1. L'utilisateur fait pivoter l'élément de fixation 9 autour de la tige 14 pour le faire passer l'élément de fixation 9 de la position libre à la position de blocage afin de bloquer la première plaque 2 et la deuxième plaque 3 en état fermé. En même temps que l'élément de fixation 9 passe dans la position de blocage, l'ergot 19 s'enclenche dans l'orifice 17 de l'élément de verrouillage 16. La filtration du fluide au travers de l'unité de filtration 4 peut débuter.

A la fin de la filtration, l'utilisateur appuie à nouveau sur l'ergot 19 de l'élément de verrouillage 16 pour libérer l'élément de fixation 9 de sa position de blocage. L'utilisateur fait pivoter l'élément de fixation 9 autour de la tige 14 pour mettre l'élément de fixation 9 en position libre. L'utilisateur fait tourner la deuxième plaque 3 pour ouvrir le dispositif de maintien 1 et permettre le retrait de l'unité de filtration 4 du dispositif de maintien 1.

Après le retrait de l'unité de filtration 4, le dispositif de maintien 1 est fermé à nouveau avec l'élément de fixation 9 dans la position de blocage comme décrit précédemment. En variante, le dispositif de maintien est laissé ouvert jusqu'à la prochaine utilisation.

Ce dispositif de maintien est réutilisable pour maintenir d'autres unités de filtration.

Selon un second aspect, l'invention concerne un ensemble comprenant un dispositif de maintien selon le premier aspect de l'invention et une unité de filtration.

En particulier, l'unité de filtration 4 comprend une enveloppe souple renfermant un milieu de filtration. Le dispositif de maintien permet particulièrement d'éviter le gonflement de l'unité de filtration lors de la filtration d'un fluide au travers de ladite unité de filtration.

La figure 6 montre un système à poches 37 comprenant une unité de filtration 4 pouvant être maintenue dans le dispositif de maintien 1 décrit ci-dessus. Le système à poches 37 comprend en outre au moins une poche de recueil du filtrat 39 correspondant à la première poche satellite du système à poche décrit ci-dessous.

Le système à poches 37 de la figure 6 est conçu pour recueillir le sang total et préparer un plasma déleucocyté et un concentré de globules rouges déleucocyté.

Le système à poches 37 comprend une poche primaire 38 destinée à recevoir le sang total collecté d'un donneur. Cette poche primaire est en communication fluidique avec d'une première part une première poche satellite 39 par l'intermédiaire d'une première tubulure 40, d'une deuxième part, une deuxième poche satellite 41 par l'intermédiaire d'une deuxième tubulure 42 et d'une troisième part, une aiguille de prélèvement 43 par l'intermédiaire d'une troisième tubulure 44. La deuxième poche satellite 41 est en outre en communication fluidique avec une troisième poche satellite 45 par l'intermédiaire d'une quatrième tubulure 46. La première tubulure 40 comprend l'unité de filtration 4 destinée à éliminer les leucocytes du plasma sanguin. La quatrième tubulure 46 comprend une autre unité de filtration 47 destinée à éliminer les leucocytes d'un concentré de globules rouges.

Dans une réalisation particulière, la première poche satellite est 39 est destinée à recueillir un plasma sanguin déleucocyté, la deuxième poche satellite 41 est destinée à recueillir un concentré de globules rouges et la troisième poche satellite 45 est destinée à recueillir un concentré de globules rouges déleucocyté.

Un exemple d'utilisation du système à poches et du dispositif de maintien est décrit ci-dessous.

Le sang d'un donneur est collecté dans la poche primaire 38 par l'intermédiaire de l'aiguille de prélèvement 43. Le système à poches 37 est placé dans une centrifugeuse afin de séparer le sang dans la poche primaire 38 en une couche inférieure comprenant le concentré de globules rouges et une couche supérieure comprenant le plasma.

Le système à poches 37 est monté sur un appareil de séparation et extraction des composants sanguins, tel que celui décrit dans le document

EP 2 869 864 A1. L'unité de filtration souple 4 est logée dans un dispositif de maintien 1 comme décrit ci-dessus.

L'appareil de séparation et extraction compresse la poche primaire 38 de sorte à transférer le plasma dans la première poche satellite 39 via l'unité de filtration 4 et le concentré de globules rouges dans la seconde poche satellite 41. La poche satellite 3.9 contient alors le plasma déleucocyté.

Finalement, le système à poches 37 est retiré de l'appareil de séparation et extraction et la deuxième poche satellite 41 est suspendue de sorte à filtrer par gravité le concentré de globules rouges au travers de l'autre unité de filtration 47. Le concentré de globules rouges déleucocyté est recueilli dans la troisième poche satellite 45.

## Revendications

1. Dispositif de maintien (1) d'une unité de filtration (4) d'un fluide biologique comprenant une première plaque (2) et une deuxième plaque (3) mobiles entre elles entre un état ouvert dans lequel la première plaque (2) et la deuxième plaque (3) sont éloignées l'une de l'autre afin de pouvoir disposer ladite unité de filtration (4) dans ledit dispositif de maintien (1), et un état fermé dans lequel la première plaque (2) et la deuxième plaque (3) sont en vis-à-vis afin de maintenir serrée ladite unité de filtration (4), ledit dispositif de maintien (1) comprenant en outre un élément de fixation (9) mobile entre une position libre dans laquelle la première plaque (2) et la deuxième plaque (3) sont libres de se déplacer entre l'état ouvert et l'état fermé et une position de blocage dans laquelle la première plaque (2) et la deuxième plaque (3) sont maintenues dans l'état fermé, **caractérisé en ce que** ce qu'il comprend en outre un élément de verrouillage (16) configuré pour maintenir l'élément de fixation (9) dans ladite position de blocage.

2. Dispositif de maintien selon la revendication 1, **caractérisé en ce que** l'élément de fixation (9) comprend une pièce présentant une fente (10) conçue pour recevoir au moins une portion de ladite première plaque (2) et ladite deuxième plaque (3) lorsqu'elles sont dans l'état fermé.

3. Dispositif de maintien selon la revendication 2, **caractérisé en ce que** ladite pièce est formée d'une première plaquette (11) et d'une deuxième plaquette (12) se faisant face et reliées entre elles par un fond (13), ladite première plaquette (11) et ladite deuxième plaquette (12) étant séparées d'une distance égale à l'épaisseur de la première plaque (2) et de la deuxième plaque (3) dans l'état fermé avec l'unité de filtration maintenue serrée dans ledit dispositif de maintien.

4. Dispositif de maintien selon l'une quelconque des revendication 1 à 3, **caractérisé en ce que** l'élément de fixation (9) est configuré pour pivoter entre ladite position de blocage et ladite position libre.

5. Dispositif de maintien selon l'une quelconque des revendications 1 à 4, **caractérisé en ce que** l'élément de fixation (9) comprend à une extrémité, une tige (14) formant un axe de rotation.

6. Dispositif de maintien selon la revendication 5, **caractérisé en ce que** la première plaque (2) comprend un pivot (15) conçu pour supporter la tige (14) de l'élément de fixation (9).

7. Dispositif de maintien selon l'une quelconque des revendications 1 à 6, **caractérisé en ce que** l'élément de verrouillage (16) comprend une partie femelle formée d'un orifice (17) et une partie mâle formée d'une languette (18) pourvue d'un ergot (19), ladite partie femelle et ladite partie mâle coopérant pour que l'ergot (19) s'engage dans l'orifice (17) de la partie femelle.

8. Dispositif de maintien selon la revendication 7, **caractérisé en ce que** la languette (18) est déformable pour permettre le déverrouillage de l'élément de verrouillage (16).

9. Dispositif de maintien selon l'une quelconque des revendications 1 à 8, **caractérisé en ce que** la première plaque (2) et la deuxième plaque (3) sont reliées l'une à l'autre par une articulation formée d'une charnière (5).

10. Dispositif de maintien selon l'une quelconque des revendications 1 à 9, **caractérisé en ce que** chacune de la première plaque (2) et de la deuxième plaque (3) comprend sur leur face interne des cavités (20,21,22,23) configurées pour recevoir des tubes d'entrée (24) et sortie (25) de l'unité de filtration (4).

11. Dispositif de maintien selon l'une quelconque des revendications 1 à 10, **caractérisé en ce que** l'une de la première plaque (2) ou la deuxième plaque (3) comprend au moins une saillie (26,27,28,29) coopérant avec au moins une encoche (30,31,32,33) prévue sur l'autre de la première plaque (2) ou deuxième plaque (3).

12. Dispositif de maintien selon l'une quelconque des revendications 1 à 11, **caractérisé en ce qu'**une face interne de la première plaque (2) ou la deuxième plaque (3) comprend une empreinte (34) de l'unité de filtration (4).

13. Ensemble comprenant un dispositif de maintien (1) selon l'une quelconque des revendications 1 à 12 et une unité de filtration (4), **caractérisé en ce que** ladite unité de filtration (4) comprend une enveloppe souple renfermant un milieu de filtration.

14. Ensemble selon la revendication 13, **caractérisé en ce que** l'unité de filtration (4) est comprise dans un système à poches (37) comprenant en outre au moins une poche de recueil du filtrat (39).

## Patentansprüche

1. Haltevorrichtung (1) für eine Filtereinheit (4) für ein biologisches Fluid, umfassend eine erste Platte (2) und eine zweite Platte (3), die zwischen einem offenen Zustand, in dem die erste Platte (2) und die zweite Platte (3) voneinander entfernt sind, um die Filtereinheit (4) in der Haltevorrichtung (1) anordnen zu können, und einem geschlossenen Zustand zueinander beweglich sind, in dem die erste Platte (2) und die zweite Platte (3) einander gegenüberliegen, um die Filtereinheit (4) fest zu halten, wobei die Haltevorrichtung (1) ferner ein Befestigungselement (9) umfasst, das zwischen einer freien Position, in der die erste Platte (2) und die zweite Platte (3) frei sind, sich zwischen dem offenen Zustand und dem geschlossenen Zustand bewegen können, und einer Verriegelungsposition beweglich ist, in der die erste Platte (2) und die zweite Platte (3) im geschlossenen Zustand gehalten werden, **dadurch gekennzeichnet, dass** sie ferner ein Verriegelungselement (16) umfasst, das konfiguriert ist, um das Befestigungselement (9) in der Verriegelungsposition zu halten.

2. Haltevorrichtung nach Anspruch 1, **dadurch gekennzeichnet, dass** das Befestigungselement (9) ein Teil umfasst, das einen Schlitz (10) aufweist, der ausgelegt ist, um mindestens einen Abschnitt der ersten Platte (2) und der zweiten Platte (3) aufzunehmen, wenn sie in dem geschlossenen Zustand sind.

3. Haltevorrichtung nach Anspruch 2, **dadurch gekennzeichnet, dass** das Teil aus einem ersten Plättchen (11) und einem zweiten Plättchen (12) gebildet ist, die einander gegenüberliegen und miteinander durch einen Boden (13) verbunden sind, wobei das erste Plättchen (11) und das zweite Plättchen (12) durch einen Abstand voneinander getrennt sind, der gleich der Dicke der ersten Platte (2) und der zweiten Platte (3) im geschlossenen Zustand mit der Filtereinheit ist, die in der Haltevorrichtung fest gehalten ist.

4. Haltevorrichtung nach einem der Ansprüche 1 bis 3, **dadurch gekennzeichnet, dass** das Befestigungselement (9) konfiguriert ist, um zwischen der Verriegelungsposition und der freien Position zu schwenken.

5. Haltevorrichtung nach einem der Ansprüche 1 bis 4, **dadurch gekennzeichnet, dass** das Befestigungselement (9) an einem Ende einen Schaft (14) umfasst, der eine Drehachse bildet.

6. Haltevorrichtung nach Anspruch 5, **dadurch gekennzeichnet, dass** die erste Platte (2) einen Drehzapfen (15) umfasst, der ausgelegt ist, um den Schaft (14) des Befestigungselements (9) zu stützen.

7. Haltevorrichtung nach einem der Ansprüche 1 bis 6, **dadurch gekennzeichnet, dass** das Verriegelungselement (16) einen aufnehmenden Teil, der aus einer Öffnung (17) gebildet ist, und einen vorstehenden Teil umfasst, der aus einer Zunge (18) gebildet ist, die mit einem Vorsprung (19) versehen ist, wobei der aufnehmenden Teil und der vorstehende Teil zusammenwirken, damit der Vorsprung (19) in die Öffnung (17) des aufnehmenden Teils eingreift.

8. Haltevorrichtung nach Anspruch 7, **dadurch gekennzeichnet, dass** die Zunge (18) verformbar ist, um das Entriegeln des Verriegelungselements (16) zu ermöglichen.

9. Haltevorrichtung nach einem der Ansprüche 1 bis 8, **dadurch gekennzeichnet, dass** die erste Platte (2) und die zweite Platte (3) durch ein Gelenk miteinander verbunden sind, das von einem Scharnier (5) gebildet wird.

10. Haltevorrichtung nach einem der Ansprüche 1 bis 9, **dadurch gekennzeichnet, dass** jede der ersten Platte (2) und der zweiten Platte (3) auf ihrer Innenseite Vertiefungen (20,21,22,23) umfasst, die zum Aufnehmen von Einlass- (24) und Auslassrohren (25) der Filtereinheit (4) konfiguriert sind.

11. Haltevorrichtung nach einem der Ansprüche 1 bis 10, **dadurch gekennzeichnet, dass** eine der ersten Platte (2) oder der zweiten Platte (3) mindestens einen Vorsprung (26,27,28,29) umfasst, der mit mindestens einer Kerbe (30,31,32,33) zusammenwirkt, die an der anderen der ersten Platte (2) oder der zweiten Platte (3) vorgesehen ist.

12. Haltevorrichtung nach einem der Ansprüche 1 bis 11, **dadurch gekennzeichnet, dass** eine Innenseite der ersten Platte (2) oder der zweiten Platte (3) eine Vertiefung (34) der Filtereinheit (4) umfasst.

13. Baugruppe, umfassend eine Haltevorrichtung (1) nach einem der Ansprüche 1 bis 12 und eine Filtereinheit (4), **dadurch gekennzeichnet, dass** die Filtereinheit (4) eine flexible Hülle umfasst, die ein Filtermedium einschließt.

14. Baugruppe nach Anspruch 13, **dadurch gekennzeichnet, dass** die Filtereinheit (4) in einem Beutelsystem (37) umfasst ist, das ferner mindestens einen Sammelbeutel des Filtrats (39) umfasst.

## Claims

1. Holding device (1) for a biological fluid filtration unit (4) comprising a first plate (2) and a second plate (3) that are movable in relation to each other between an open state in which the first plate (2) and the second plate (3) are remote from each other in order to be able to arrange said filtration unit (4) in said holding device (1), and a closed state in which the first plate (2) and the second plate (3) are facing each other in order to hold said filtration unit (4) clamped, said holding device (1) further comprising a fixing element (9) movable between a free position in which the first plate (2) and the second plate (3) are free to move between the open state and the closed state and a blocking position in which the first plate (2) and the second plate (3) are held in the closed state, **characterized in that** it further comprises a locking element (16) configured to hold the fixing element (9) in said blocking position.

2. Holding device according to claim 1, **characterized in that** the fixing element (9) comprises a part having a slot (10) designed to receive at least a portion of said first plate (2) and said second plate (3) when they are in the closed state.

3. Holding device according to claim 2, **characterized in that** said part is formed by a first small plate (11) and a second small plate (12) facing each other and connected to each other by a bottom (13), said first plate (11) and said second plate (12) being separated by a distance equal to the thickness of the first plate (2) and the second plate (3) in the closed state with the filtration unit held clamped in said holding device.

4. Holding device according to any one of claims 1 to 3, **characterized in that** the fixing element (9) is configured to pivot between said blocking position and said free position.

5. Holding device according to any one of claims 1 to 4, **characterized in that** the fixing element (9) comprises, at one end, a rod (14) forming an axis of rotation.

6. Holding device according to claim 5, **characterized in that** the first plate (2) comprises a pivot (15) designed to support the rod (14) of the fixing element (9).

7. Holding device according to any one of claims 1 to 6, **characterized in that** the locking element (16) comprises a female part formed by an orifice (17) and a male part formed by a tongue (18) provided with a lug (19), said female part and said male part cooperating so that the lug (19) engages in the orifice (17) of the female part.

8. Holding device according to claim 7, **characterized in that** the tongue (18) is deformable to allow unlocking of the locking element (16).

9. Holding device according to any one of claims 1 to 8, **characterized in that** the first plate (2) and the second plate (3) are connected to each other by a joint formed by a hinge (5).

10. Holding device according to any one of claims 1 to 9, **characterized in that** each of the first plate (2) and the second plate (3) comprises on their inner face cavities (20, 21, 22, 23) configured to receive inlet (24) and outlet (25) tubes of the filtration unit (4).

11. Holding device according to any one of claims 1 to 10, **characterized in that** one of the first plate (2) or the second plate (3) comprises at least one projection (26, 27, 28, 29) cooperating with at least one notch (30, 31, 32, 33) provided on the other of the first plate (2) or second plate (3).

12. Holding device according to any one of claims 1 to 11, **characterized in that** an inner face of the first plate (2) or the second plate (3) comprises an imprint (34) of the filtration unit (4).

13. Assembly comprising a holding device (1) according to any one of claims 1 to 12 and a filtration unit (4), **characterized in that** said filtration unit (4) comprises a flexible envelope enclosing a filtration medium.

14. Assembly according to claim 13, **characterized in that** the filtration unit (4) is comprised in a bag system (37) further comprising at least one filtrate collection bag (39).
